# EUROPEAN PATENT SPECIFICATION

(11) **EP 3 897 398 B1**
(45) Date of publication and mention of the grant of the patent: **01.02.2023**
(21) Application number: 19829202.1
(22) Date of filing: 20.12.2019
(51) Int. Cl.: A61B 17/02, A61F 9/00

(54) **DEVICE FOR SEPARATING A PATIENT'S EYELID**
VORRICHTUNG ZUR TRENNUNG EINES AUGENLIDES EINES PATIENTEN
DISPOSITIF DE SÉPARATION D'UNE PAUPIÈRE D'UN PATIENT

(30) Priority: 21.12.2018 ES 201831263
(43) Date of publication of application: 27.10.2021
(73) Proprietor: Administración General de la Comunidad Autónoma de Euskadi, 01010 Vitoria-Gasteiz, Álava (ES); Mizar Additive Manufacturing, S.L.U., 01510 Miñano/Álava (ES)
(72) Inventor: GARAY ARAMBURU, Gonzaga, 01004 VITORIA-GASTEIZ/Álava (ES); SANCHEZ ARIZMENDIARRIETA, Xabier, 01510 MIÑANO/Álava (ES)
(74) Representative: Pons
(86) International application number: PCT/EP2019/086689
(87) International publication number: WO 2020/127978

(56) References cited:
- WO-A1-2017/014630
- US-A- 5 762 606
- US-A1- 2010 241 102
- US-A1- 2017 042 529

## Description

### OBJECT OF THE INVENTION

The present application relates to a device for separating the eyelid, locating the injection site and orienting the needle in procedures for the intravitreal injection of medicinal substances.

### BACKGROUND OF THE INVENTION

The use of devices for separating the eyelid in procedures for intravitreal injection is known. Said intravitreal injections are necessary in patients with pathologies such as macular degeneration, diabetes and other retinal diseases.

In the injection process today, devices for separating the eyelid, called blepharostats, and different types of markers, are used. The injection must be performed at 3.5 mm in the case of patients operated on for cataracts and at 4 mm in those who are not.

In the documents known in the state of the art by the applicant, the method for separating the eyelid consists of gently pushing and thereby separating the eyelid to proceed with the intravitreal injection of medicinal substances. This gives rise to an unsafe process because it is being held by slight pressure or pushing without making it impossible for the patient to close his/her eye throughout the process.

Additionally, the devices known by the applicant have holes with a concave surface, limiting their use to a certain type of syringes or needles having specific shapes.

The known devices comprise holes with constant distances from the hole to the center of the vitreous cavity. However, for patients operated on for cataracts this distance must be less, more particularly about 3.5 mm with respect to the 4 mm for other patients. Therefore, it has become necessary to manufacture these devices with different distances and geometries, depending on each patient, making the large-scale production of disposable devices difficult and expensive.

The "InVitria 4" injection assistant by InVitria^{®}, designed by Arnaldo Gonçalves, is based on a disposable device for intravitreal injections configured for obtaining a position with a fixed injection angle. The proposed method of fixing of the upper eyelid is based on pushing the device towards the eye and turning, with the eye thereby being exposed to receive the injection through the orifice designed to that end. However, the InVitria^{®} device does not fix the eyelid by pulling, whereby making it less safe in the event of sudden movements, in addition to the discomfort it causes the patient. The "InVitria 4" injection assistant has a single injection distance of 3.5 mm with respect to the corneoscleral limbus. That is valid for patients operated on for cataracts, however, said distance should preferably be 4 mm for other patients.

Patent application EP2109425 (A1), the applicant for which is Arnaldo Gonçalves, discloses a device for the intraocular administration of a substance by means of a hypodermic needle. It comprises a support element to be placed on the eye and a hole in said support, arranged perpendicular to the surface of the eye and configured for introducing the hypodermic needle. However, the proposed device does not comprise pulling means for separating the upper eyelid, the proposed method of fixing said eyelid is based on the pressure applied by the support on the surface of the eye.

Patent application EP2017266045 (A1), the applicant for which is VISIONITY OY [Fl], discloses an ocular therapeutic tool including a hollow body connected to a base, where said base is adapted for being placed on the surface of the eye. The invention proposed in this application also comprises an injection guide connectable to the hollow body and adapted to receive a needle. However, the proposed invention is not a one-piece tool, with the drawback of the brittleness involved with these structures. Furthermore, the tool does not comprise shoulders or other elements the purpose of which is to separate the eyelid either.

Patent application AU2015201289 (A1), the applicant for which is INST NAT SANTE RECH MED, discloses a device for injecting an ocular product, comprising an injection needle, a support configured for carrying out 5 movements of the needle; and wherein said device comprises a locating mark fixed in the support which is located in contact with the region of the eye in which the injection takes place. This device primarily relates to a device where the injection needle has freedom of movement; however, it does not propose an additional method based on shoulders or hooks to pull on the patient's upper eyelid.

United States patent application US2014094752 (A1), with applicant HILES JOHN [GB]; SANOFI AVENTIS DEUTSCHLAND [DE], describes a guide device for intraocular injection, comprising a base having an inner surface and an outer surface. Said base in turn comprises a handle on the outer surface and a through hole in both surfaces; and where the inner surface is adapted for being placed in contact with the eyeball. This invention does not propose any method of separating the eyelid by pulling, nor does it provide any structural differences for varying the distance with respect to the corneoscleral limbus into which the injection is to be made.

US 2010/241102 discloses a device for separating a patient's eyelid in procedures for the intravitreal injection of medicinal substances, comprising: - a distal sector further comprising a body and a shoulder, wherein the body comprises a curved front face intended for contacting the corneoscleral limbus of a patient's eye and wherein said body has at least one orifice adapted for introducing a needle of a syringe; - an elongated handle extending from the body and located at the opposite end of the distal sector of the device; the shoulder is hook-shaped for contacting the patient's upper eyelid, and wherein the handle forms a longitudinal geometry from its distal sector to the handle.

WO 2017/014630 discloses a device with a syringe assembly, a tubular housing and a positioning element.

The present invention seeks to solve some of the problems mentioned in the state of the art.

More specifically, the present invention describes a device which facilitates the intravitreal injection process by locating the injection point, guiding the injection needle towards the center of the vitreous cavity, making it safer for the patient and simplifying the process with fewer parts with respect to devices known in the state of the art.

More particularly, the present invention discloses a device for separating a patient's eyelid in procedures for the intravitreal injection of medicinal substances, comprising:
- a distal sector further comprising a body and a shoulder, wherein the body comprises a curved front face intended for contacting the corneoscleral limbus of a patient's eye and wherein said body has at least one orifice adapted for introducing a needle of a syringe;
- an elongated handle extending from the body of the distal end and located at the opposite end of the distal sector of the device;
wherein the shoulder is hook-shaped for contacting the patient's upper eyelid and wherein the device has a longitudinal geometry from its distal sector to the handle, suitable for pulling on the patient's upper eyelid by means of the hook-shaped shoulder with the patient in a supine decubitus position.

By means of the technical features mentioned above, the patient's upper eyelid is held by pulling, which allows for a much safer procedure.

The device is preferably one-piece, that is, it forms one body consisting of a single part, allowing for a more robust device, thus avoiding mechanisms that can fracture or come apart, or parts that may be lost. The device fundamentally prevents a possible, and dangerous, fracturing between parts during the intravitreal injection.

More preferably, the device forms a curvilinear longitudinal body from the distal sector to the handle, which allows comfortably pulling by lever means with the fingers of one hand.

Preferably the orifice for introducing the syringe is straight, which allows using different types of needles with different types of shapes.

The device has a viewing opening in the body of the distal sector which allows examining the behavior of the eye while the intravitreal injection is being applied.

The distal end adapted for contacting the corneoscleral limbus of the patient's eye can be arcuate.

Preferably, the orifice comprises a distance of about 4 mm to the corneoscleral junction or limbus. Said distance is suitable for patients who have not been operated on for cataracts.

More preferably, the device has a second orifice with a different distance to the corneoscleral junction or limbus with respect to the distance of the first orifice.

Preferably, said distance from the second orifice is about 3.5 mm, said distance being suitable for patients who have been operated on for cataracts.

### DESCRIPTION OF THE DRAWINGS

To complement the description that is being made and for the purpose of helping to better understand the features of the invention according to a preferred practical exemplary embodiment thereof, a set of drawings is attached as an integral part of said description in which the following is depicted in an illustrative and nonlimiting manner:
Figure 1 shows a rear perspective view of the device according to a preferred embodiment of the present invention.
Figure 2 shows an enlarged view of the distal sector of the device for separating the eyelid according to the preferred embodiment, where the through holes, the viewing opening and the hook-shaped shoulder of the device for separating the eyelid are clearly shown in the distal sector.
Figure 3 shows a side view of the device according to the preferred embodiment of the invention.
Figure 4 shows a rear view of the distal sector according to the preferred embodiment of the device for separating a patient's eyelid for intravitreal injections, where the body in the distal sector comprising through holes is clearly shown, and the asymmetry in the distance to the center of the vitreous cavity can furthermore be observed.

### PREFERRED EMBODIMENT OF THE INVENTION

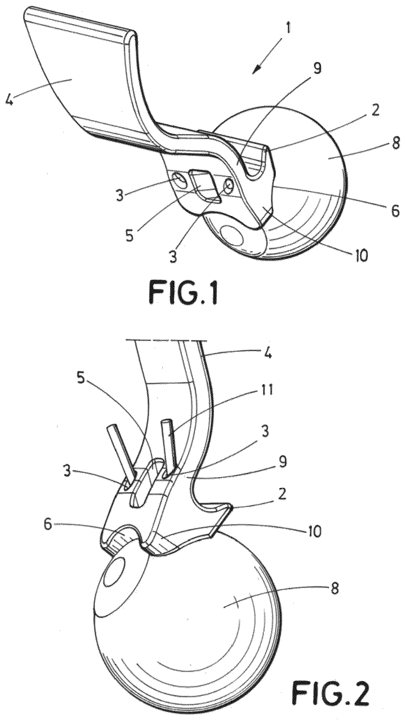

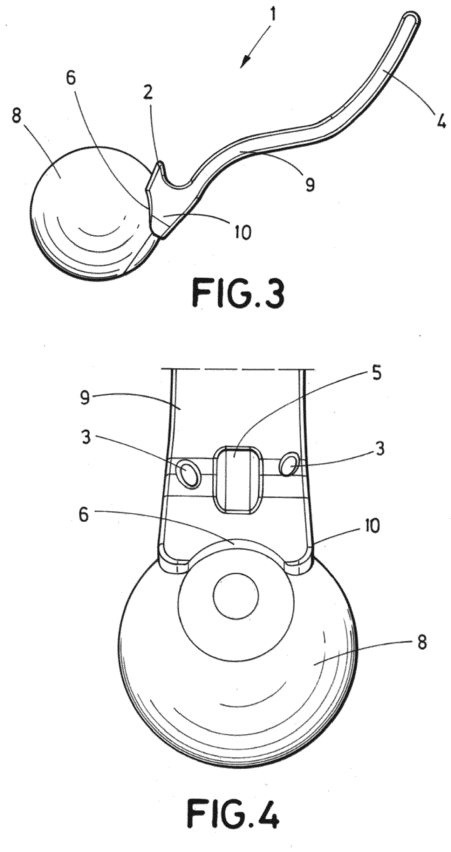

Figure 1 shows a perspective view of a preferred embodiment of the invention of a device (1) for separating a patient's eyelid in procedures for the intravitreal injection of medicinal substances, comprising a distal sector (9) geometrically adapted for contacting the corneoscleral limbus of the patient's eye (8), said distal sector (9) further comprising a body (10) and a shoulder (2). The body (10) comprises two holes (3) for the purpose of guiding a needle of a syringe for intravitreal injection to the suitable location of the center of the intravitreal cavity, each hole in the preferred embodiment having a straight and smooth surface open for passage. Each hole has different distances to the corneoscleral junction or limbus of the patient's eye (8). One of the holes (3) has a distance of 3.5 mm, intended for patients who have had cataracts, and the other hole (3) has a distance of 4 mm for patients who have not had cataracts (see Figure 4). The body further comprises a curved front face (6).

Figure 2 shows an enlarged view of the distal sector (9) supported on the corneoscleral limbus of the eye (8). As shown in Figure 2, said distal sector (9) has a hook-shaped shoulder (2) for contacting the patient's upper eyelid. The distal sector further comprises a viewing opening (5) that transversely goes through the body (10) and allows examining the behavior of the eye while the intravitreal injection is being applied, fundamentally to exclude blood vessels.

The device (1) comprises an elongated handle (4) extending from the body (10) and located at the opposite end of the distal sector (9) of the device (1). The handle is ergonomically adapted to be held with the fingers and pulling on the patient's upper eyelid by means of the hook-shaped shoulder (2) with the patient in a supine decubitus position.

Figure 3 shows a side view of the device (1) according to the preferred embodiment of the invention. Said device (1) in the described preferred embodiment forms a body that is substantially curvilinear in its longitudinal direction, that is from the handle (4) to its distal sector (9). It preferably forms a curvilinear body with an odd symmetric function, as shown in Figure 3. Application in the patient in supine decubitus position is thereby facilitated, and comfortably pulling on the upper eyelid by means of a lever with the fingers of one hand is also facilitated.

It should be noted that the device (1) in the described preferred embodiment comprises only one part, allowing for a more robust device, thus avoiding mechanisms that can fracture or come apart, or parts that may be lost. The device fundamentally prevents a possible, and dangerous, fracturing between parts during the intravitreal injection.

Figure 4 shows a rear view of the device according to the preferred embodiment of the where the through holes (3) in the body (10) and the asymmetry in the distance to the corneoscleral limbus, more specifically a hole (3) at about 3.5 mm for patients who have been operated on for cataracts and the other hole (3), with an offset of 0.5 mm, at 4 mm from the center of the vitreous cavity, are clearly shown.

## Claims

1. A device (1) for separating a patient's eyelid in procedures for the intravitreal injection of medicinal substances, comprising:
- a distal sector (9) further comprising a body (10) and a shoulder (2), wherein the body comprises a curved front face (6) intended for contacting the corneoscleral limbus of a patient's eye (8) and wherein said body (10) has at least one orifice (3) adapted for introducing a needle of a syringe (11);
- an elongated handle (4) extending from the body (10) and located at the opposite end of the distal sector (9) of the device (1);
the shoulder (2) is hook-shaped for contacting the patient's upper eyelid, and wherein the handle (4) forms a longitudinal geometry from its distal sector (9) to the handle (4), **characterized in that** the device is suitable for pulling on the patient's upper eyelid by means of the hook-shaped shoulder (2) with the patient in a supine decubitus position, wherein the body (10) further comprises a viewing opening (5) that transversely goes through the body (10) located in the distal section (9) and upwardly to the curved front face (6), which allows to examine the behavior of the eye while applying the intravitreal injection.

2. The device according to claim 1, **characterized in that** the device (1) forms one body consisting of a single part.

3. The device according to any of the preceding claims, **characterized in that** it forms a curvilinear longitudinal body from the distal sector (9) to the handle (4).

4. The device according to any of the preceding claims, **characterized in that** the curved front face (6) of the body (10) in the distal sector (9) is arcuate.

5. The device according to any of the preceding claims, **characterized in that** at least one orifice (3) is directed towards the center of the vitreous cavity.

6. The device according to any of the preceding claims, **characterized in that** the device has a second orifice (3) with a distance to the corneoscleral limbus different from that of the first orifice.

7. The device according to claim 5, **characterized in that** the first orifice comprises a distance of about 4 mm to the corneoscleral limbus.

8. The device according to claim 6, **characterized in that** the distance from the second orifice is about 3.5 mm.

## Patentansprüche

1. Vorrichtung (1) zum Separieren des Augenlids eines Patienten bei Verfahren zur intravitrealen Injektion von Arzneimitteln, umfassend:
- einen distalen Abschnitt (9), der ferner einen Körper (10) und eine Schulter (2) umfasst, wobei der Körper eine gekrümmte Vorderseite (6) umfasst, die dazu bestimmt ist, um mit dem Limbus corneae des Auges (8) eines Patienten in Kontakt zu treten, und wobei der Körper (10) mindestens eine Öffnung (3) aufweist, die zum Einführen einer Nadel einer Spritze (11) angepasst ist;
- einen länglichen Griff (4), der sich vom Körper (10) erstreckt und sich am gegenüberliegenden Ende des distalen Abschnitts (9) der Vorrichtung (1) befindet;
wobei die Schulter (2) hakenförmig ist, um mit dem oberen Augenlid des Patienten in Kontakt zu treten, und wobei der Griff (4) eine Längsgeometrie von seinem distalen Abschnitt (9) zum Griff (4) bildet, **dadurch gekennzeichnet, dass** die Vorrichtung geeignet ist, um mittels der hakenförmigen Schulter (2) am Oberlid des Patienten zu ziehen, wenn sich der Patient in Rückenlage befindet, wobei der Körper (10) ferner eine Sichtöffnung (5) aufweist, die quer durch den Körper (10), der sich im distalen Abschnitt (9) befindet, und nach oben zur gekrümmten Vorderseite (6) verläuft, wodurch das Verhalten des Auges während der Verabreichung der intravitrealen Injektion untersucht werden kann.

2. Vorrichtung nach Anspruch 1, **dadurch gekennzeichnet, dass** die Vorrichtung (1) einen Körper bildet, der aus einem einzigen Teil besteht.

3. Vorrichtung nach einem der vorhergehenden Ansprüche, **dadurch gekennzeichnet, dass** sie vom distalen Abschnitt (9) bis zum Griff (4) einen kurvilinearen Längskörper bildet.

4. Vorrichtung nach einem der vorhergehenden Ansprüche, **dadurch gekennzeichnet, dass** die gekrümmte Vorderseite (6) des Körpers (10) im distalen Abschnitt (9) bogenförmig ist.

5. Vorrichtung nach einem der vorhergehenden Ansprüche, **dadurch gekennzeichnet, dass** mindestens eine Öffnung (3) auf die Mitte des Glaskörperraums gerichtet ist.

6. Vorrichtung nach einem der vorhergehenden Ansprüche, **dadurch gekennzeichnet, dass** die Vorrichtung eine zweite Öffnung (3) mit einem Abstand zum Limbus corneae, der sich von dem der ersten Öffnung unterscheidet, aufweist.

7. Vorrichtung nach Anspruch 5, **dadurch gekennzeichnet, dass** die erste Öffnung einen Abstand von etwa 4 mm zum Limbus corneae aufweist.

8. Vorrichtung nach Anspruch 6, **dadurch gekennzeichnet, dass** der Abstand von der zweiten Öffnung etwa 3,5 mm beträgt.

## Revendications

1. Dispositif (1) pour séparer la paupière d'un patient dans des procédures pour l'injection intravitréenne de substances médicamenteuses, comprenant :
- un secteur distal (9) comprenant en outre un corps (10) et un épaulement (2), dans lequel le corps comprend une face avant incurvée (6) destinée à venir en contact avec le limbe cornéoscléral de l'oeil d'un patient (8) et dans lequel ledit corps (10) présente au moins un orifice (3) conçu pour l'introduction d'une aiguille d'une seringue (11) ;
- une poignée allongée (4) s'étendant depuis le corps (10) et située à l'extrémité opposée du secteur distal (9) du dispositif (1) ;
l'épaulement (2) est en forme de crochet pour venir en contact avec la paupière supérieure du patient, et dans lequel la poignée (4) forme une géométrie longitudinale depuis son secteur distal (9) jusqu'à la poignée (4), **caractérisé en ce que** le dispositif est apte à tirer sur la paupière supérieure du patient au moyen de l'épaulement en forme de crochet (2) avec le patient dans une position de décubitus dorsal, dans lequel le corps (10) comprend en outre une ouverture de visualisation (5) qui traverse transversalement le corps (10) situé dans la section distale (9) et vers le haut jusqu'à la face avant incurvée (6), ce qui permet d'examiner le comportement de l'œil lors de l'application de l'injection intravitréenne.

2. Dispositif selon la revendication 1, **caractérisé en ce que** le dispositif (1) forme un corps constitué d'une seule pièce.

3. Dispositif selon l'une quelconque des revendications précédentes, **caractérisé en ce qu'**il forme un corps longitudinal curviligne depuis le secteur distal (9) jusqu'à la poignée (4).

4. Dispositif selon l'une quelconque des revendications précédentes, **caractérisé en ce que** la face avant incurvée (6) du corps (10) dans le secteur distal (9) est en arc de cercle.

5. Dispositif selon l'une quelconque des revendications précédentes, **caractérisé en ce qu'**au moins un orifice (3) est dirigé vers le centre de la cavité vitréenne.

6. Dispositif selon l'une quelconque des revendications précédentes, **caractérisé en ce que** le dispositif a un second orifice (3) avec une distance au limbe cornéoscléral différente de celle du premier orifice.

7. Dispositif selon la revendication 5, **caractérisé en ce que** le premier orifice comprend une distance d'environ 4 mm au limbe cornéoscléral.

8. Dispositif selon la revendication 6, **caractérisé en ce que** la distance du second orifice est d'environ 3,5 mm.
